# EUROPÄISCHE PATENTSCHRIFT

(11) **EP 1 280 755 B1**
(45) Veröffentlichungstag und Bekanntmachung des Hinweises auf die Patenterteilung: **23.08.2006**
(21) Anmeldenummer: 01956433.5
(22) Anmeldetag: 11.05.2001
(51) Int. Cl.: C07C 45/50, C07C 253/30, C07C 67/347

(54) **VERFAHREN ZUR HERSTELLUNG VON ALDEHYDEN**
METHOD FOR PRODUCING ALDEHYDES
PROCEDE POUR LA PRODUCTION D'ALDEHYDES

(30) Priorität: 12.05.2000 DE 10023470
(43) Veröffentlichungstag der Anmeldung: 05.02.2003
(73) Patentinhaber: BASF Aktiengesellschaft, 67056 Ludwigshafen (DE)
(72) Erfinder: AHLERS, Wolfgang, 67549 Worms (DE); SLANY, Michael, 67281 Kirchheim (DE)
(74) Vertreter: Kinzebach, Werner
(86) Internationale Anmeldenummer: PCT/EP2001/005406
(87) Internationale Veröffentlichungsnummer: WO 2001/085662

(56) Entgegenhaltungen:
- WO-A-98/42717

## Beschreibung

Die vorliegende Erfindung betrifft die Verwendung eines zweizähnigen Disphospinliganden und einer Quelle eines Metalls der VIII. Nebengruppe in einem Verfahren zur selektiven Herstellung von n-Aldehyden, bei dem man wenigstens eine Verbindung mit einer Vinyl- oder Vinylidengruppe in Gegenwart der Quelle eines Metalls der VIII. Nebengruppe und des zweizähnigen Diphosphin-Liganden mit Kohlenmonoxid und Wasserstoff umsetzt.

Die Hydroformylierung oder Oxosynthese ist ein wichtiges großtechnisches Verfahren und dient der Herstellung von Aldehyden durch Umsetzung von ethylenisch ungesättigten Verbindungen mit Kohlenmonoxid und Wasserstoff. Die Reaktion selbst ist stark exotherm und läuft im Allgemeinen unter erhöhtem Druck und bei erhöhten Temperaturen in Gegenwart von Katalysatoren ab. Als Katalysatoren werden meist Metalle der VIII. Nebengruppe des Periodensystems, insbesondere Cobalt-, Rhodium-, Iridium-, Ruthenium, Palladium- oder Platinverbindungen bzw. -komplexe eingesetzt, die zur Aktivitäts- und/oder Selektivitätsbeeinflussung mit stickstoff- oder phosporhaltigen Liganden modifiziert sein können.

Bei unsymmetrischen ethylenisch ungesättigten Verbindungen führen die beiden möglichen Orientierungen der Kohlenmonoxid-Anlagerung an die C-C-Doppelbindung zu unterschiedlichen Aldehyden. In der Regel wird daher ein Gemisch isomerer Aldehyde erhalten, wie dies nachstehend veranschaulicht ist.

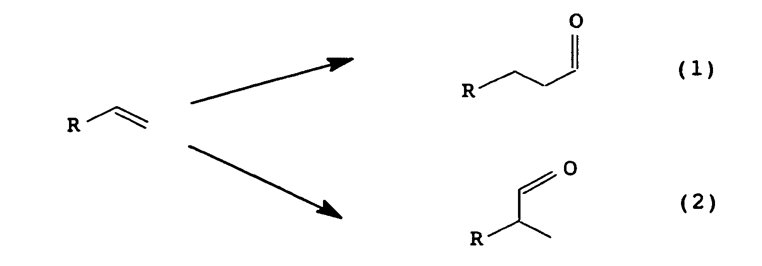

Die Verbindung (1) wird häufig als n-Aldehyd, die Verbindung (2) als Iso-Aldehyd bezeichnet.

Aufgrund der im Allgemeinen wesentlich größeren technischen Bedeutung der n-Aldehyde gegenüber den Iso-Aldehyden wird eine Optimierung der Hydroformylierungskatalysatoren und -bedingungen zur Erzielung einer möglichst großen n-Selektivität, d. h. eines möglichst hohen Verhältnisses von n-Aldehyd zu Iso-Aldehyd in den Produktaldehyden, angestrebt.

Die WO 98/42717 beschreibt Carbonylierungsreaktionen in Gegenwart eines Carbonylierungskatalysators, der ein Diphosphin umfasst, wovon wenigstens ein Phosphoratom Bestandteil einer 2-Phosphatricyclo[3.3.1.1{3,7}]decylgruppe ist. Zu den beschriebenen Carbonylierungsreaktionen zählen auch Hydroformylierungen. Obgleich die WO 98/42717 angibt, dass zur Herstellung des dort beschriebenen Katalysatorsystems der Ligand relativ zum Metallkation im Allgemeinen im Überschuß eingesetzt wird, ist hinsichtlich der Ligandenmenge, die während der Carbonylierungsreaktion vorliegt, nichts gesagt. In den Hydroformylierungsbeispielen der WO 98/42717 werden molare Verhältnisse von Diphosphinligand zu Rhodiummetall von 1:1,2 (Beispiel 9), 1:1 (Beispiel 10) bzw. 1:2 (Beispiel 11) verwendet. Bei der Hydroformylierung von Propen wird ein etwa äquimolares Gemisch von Butanal und 2-Methylpropanal erhalten.

Der vorliegenden Erfindung liegt die Aufgabe zugrunde, ein Verfahren zur Herstellung von Aldehyden durch Hydroformylierung von Verbindungen mit wenigstens einer Vinyl- oder Vinylidengruppe anzugeben, das eine möglichst hohe n-Selektivität aufweist.

Erfindungsgemäß wird diese Aufgabe gelöst durch die Verwendung eines zweizähnigen Diphosphin-Liganden der allgemeinen Formel I 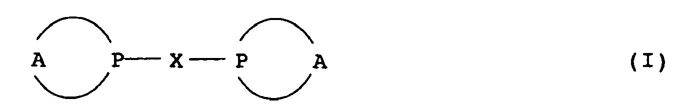 worin
- A: zusammen mit dem Phosphoratom, an das es gebunden ist, jeweils einen 2-Phospha-tricyclo[3.3.1.1{3,7}]decyl rest bildet, in dem gegebenenfalls ein oder mehrere nichtbenachbarte Kohlenstoffatome durch Sauerstoffatome ersetzt sind und der gegebenenfalls substituiert ist, und
- X: für eine verbrückende Kette mit 1 bis 10 Atomen steht,
und einer Quelle eines Metalls der VIII. Nebengruppe in einem molaren Verhältnis von wenigstens 5 zur selektiven Herstellung von n-Aldehyden in einem Verfahren, bei dem man wenigstens eine Verbindung mit einer Vinyl- oder Vinylidengruppe in Gegenwart der Quelle eines Metalls der VIII. Nebengruppe und des zweizähnigen Diphosphin-Liganden mit Kohlenmonoxid und Wasserstoff umsetzt.

Das molare Verhältnis von Disphospin-Ligand zu Metall beträgt erfindungsgemäß wenigstens 5, vorzugsweise wenigstens 8 und insbesondere beträgt es wenigstens etwa 10. Das molare Verhältnis ist im Allgemeinen kleiner als etwa 50, meistens kleiner als etwa 20.

Tricyclo[3.3.1.1{3,7}]decan ist auch unter dem Trivialnamen "Adamantan" bekannt. In dem 2-Phospha-tricyclo[3.3.1.1{3,7}]decylrest des erfindungsgemäßen verwendeten Liganden können ein oder mehrere nicht benachbarte Kohlenstoffatome, die vorzugsweise nicht in Nachbarstellung zum Phosphoratom stehen, durch Sauerstoffatome ersetzt sein. Vorzugsweise sind die Kohlenstoffatome in den Positionen 6, 9 und 10 durch Sauerstoffatome ersetzt.

Der 2-Phospha-tricyclo[3.3.1.1{3,7}]decylrest kann an einem oder mehreren seiner Kohlenstoffatome Substituenten tragen. Vorzugsweise tragen ein oder mehrere Kohlenstoffatome an den Positionen 1, 3, 5 und/oder 7, insbesondere alle Kohlenstoffe an den Positionen 1, 3, 5 und 7 Substituenten, die vorzugsweise identisch sind. Geeignete Substituenten sind z. B. Alkyl, Cycloalkyl, Halogenalkyl, Aryl oder Aralkyl. Die Kohlenstoffatome an den Positionen 4 und/oder 8 können einen oder zwei Substituenten, wie C₁-C₄-Alkyl oder Halogenatome, insbesondere Fluoratome, tragen.

Die beiden in dem erfindungsgemäß zu verwendenden Diphosphin-Liganden enthaltenen 2-Phospha-tricyclo[3.3.1.1{3,7}]decylreste können gleich oder unterschiedlich substituiert sein. Je nach Substitutionsmuster können die Diphosphine in Form von Diastereomeren vorliegen. In der Regel sind sowohl die Diastereomerengemische als auch die reinen Diastereomere für die erfindungsgemäßen Zwecke geeignet.

X steht für eine verbrückende Kette mit 1 bis 10 Atomen, vorzugsweise 2 bis 4 Atomen, insbesondere 3 Atomen. Vorzugsweise steht X für eine C₁- bis C₁₀-Alkylenbrücke, die eine, zwei, drei oder vier Doppelbindungen aufweisen kann und/oder durch ein, zwei oder drei nichtbenachbarte Heteroatome unterbrochen sein kann und/oder ein-, zwei- oder dreifach mit einem gesättigten oder ungesättigten 3- bis 7-gliedrigen Carbocyclus oder Heterocyclus anelliert sein kann.

Bei den anellierten ungesättigten Carbocyclen des Restes X handelt es sich bevorzugt um Benzol oder Naphthalin, insbesondere um Benzol. Anellierte Benzolringe sind vorzugsweise unsubstituiert oder weisen 1, 2 oder 3, insbesondere 1 oder 2 Substituenten auf, die ausgewählt sind unter Alkyl, Alkoxy, Halogen, Halogenalkyl, Nitro, Carboxyl, Alkoxycarbonyl, und Cyano. Anellierte gesättigte Carbocyclen sind vorzugsweise Cyclopropyl, Cyclobutyl, Cyclopentyl oder Cyclohexyl.

Wenn die Alkylenbrücke des Restes X durch Heteroatome unterbrochen ist, so sind diese bevorzugt ausgewählt unter Sauerstoff, Schwefel oder Stickstoff.

Bevorzugte Reste X sind ausgewählt unter

- (CH₂)ₓ -

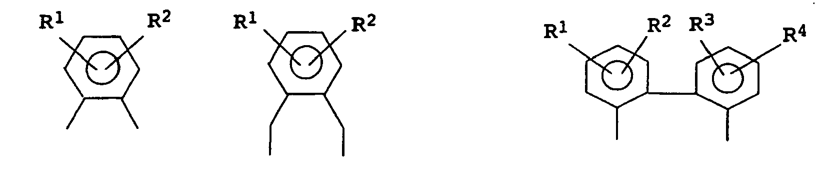

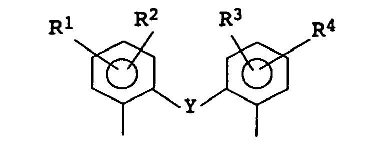

worin x für ganze Zahl von 1 bis 10, vorzugsweise 2 bis 4 steht,
Y für O, S, NR⁵ steht, wobei R⁵ für Alkyl, Cycloalkyl oder Aryl steht
oder Y für C₁-C₃-Alkylenbrücke steht, die eine Doppelbindung und/oder einen Alkyl-, Cycloalkyl- oder Arylsubstituenten aufweisen kann,
oder Y für eine C₂-C₃-Alkylenbrücke steht, die durch O, S oder NR⁵ unterbrochen ist,
R¹, R², R³ und R⁴ unabhängig voneinander für Wasserstoff, Alkyl, Cycloalkyl, Halogenalkyl, Aryl, Alkoxy, Aryloxy, Aralkyloxy, Halogen, Nitro, Alkoxycarbonyl oder Cyano stehen.

Besonders bevorzugt steht X für Propylen.

Vorzugsweise steht A zusammen mit dem Phosphoratom, an das es gebunden ist, für eine Gruppe der allgemeinen Formel II 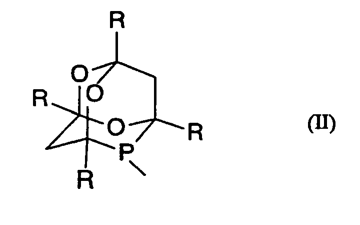 worin die Reste R unabhängig voneinander für Alkyl, Cycloalkyl, Halogenalkyl, Aryl oder Aralkyl stehen.

Im Rahmen der vorliegenden Anmeldung bedeuten, soweit nicht anders angegeben, die Ausdrücke

"Alkyl" geradkettiges oder verzweigtes Alkyl, vorzugsweise C₁-C₂₀-Alkyl, insbesondere C₁-C₈-Alkyl, besonders bevorzugt C₁-C₄₋Alkyl. Beispiele für Alkylgruppen sind insbesondere Methyl, Ethyl, Propyl, Isopropyl, n-Butyl, 2-Butyl, s-Butyl, t-Butyl, n-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl, n-Heptyl, 2-Heptyl, 3-Heptyl, 2-Ethylpentyl, 1-Propylbutyl, Octyl;

"Cycloalkyl" vorzugsweise C₅-C₇-Cycloalkyl, wie Cyclopentyl, Cyclohexyl oder Cycloheptyl;

"Halogenalkyl" vorzugsweise C₁-C₄-Halogenalkyl, d. h. einen C₁-C₄-Alkylrest, der partiell oder vollständig durch Fluor, Chlor, Brom und/oder Iod substituiert ist, wie Chlormethyl, Dichlormethyl, Trichlormethyl, Fluormethyl, Difluormethyl, Trifluormethyl, Chlorfluormethyl, Dichlorfluormethyl, Chlordifluormethyl, 2-Fluorethyl, 2-Chlorethyl, 2-Bromethyl, 2-Iodethyl, 2,2-Difluorethyl, 2,2,2-Trifluorethyl, 2-Chlor-2-fluorethyl, 2,2-Difluorethyl, 2,2-Dichlor-2-fluorethyl, 2,2,2-Trichlorethyl, Pentafluorethyl, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl und Nonafluorbutyl;

"Aryl" vorzugsweise C₆-C₁₆-Aryl, wie Phenyl, Tolyl, Xylyl, Mesityl, Naphthyl, Anthracenyl, Phenantrenyl, Naphthacenyl; insbesondere Phenyl oder Naphthyl;

"Aralkyl" vorzugsweise C₇-C₂₀-Aralkyl, insbesondere Phenyl-C₁-C₄-alkyl, wie Benzyl oder Phenethyl;

"Alkoxy" vorzugsweise C₁-C₂₀-Alkoxy mit einer Alkylgruppe vorzugsweise wie vorstehend definiert;

"Cycloalkyloxy" vorzugsweise C₅-C₇-Cycloalkyloxy mit einer Cycloalkylgruppe vorzugsweise wie vorstehend definiert;

"Aryloxy" vorzugsweise C₇-C₁₆-Aryloxy mit einer Arylgruppe vorzugsweise wie vorstehend definiert;

"Aralkyloxy" vorzugsweise C₇-C₂₀-Aralkyloxy mit einer Aralkylgruppe vorzugsweise wie vorstehend definiert;
und "Halogen" Fluor, Chlor, Brom oder Iod, vorzugsweise Fluor oder Chlor;

Besonders bevorzugt stehen die Reste R unabhängig voneinander für C₁-C₄-Alkyl, C₁-C₄-Halogenalkyl oder Phenyl, insbesondere Methyl, t-Butyl, Trifluormethyl oder Phenyl.

Besonders bevorzugte Liganden sind unter 1,2-P,P'-Di(2-phospha-1,3,5,7-tetramethyl-6,9,10-trioxa-tricyclo[3.3.1.1{3,7}]decyl)-ethan, 1,3-P,P'-Di(2-phospha-1,3,5,7-tetramethyl-6,9,10-trioxa-tricyclo[3.3.1.1{3,7}]decyl)-propan und 1,6-P,P'-Di(2-phospha-1,3,5,7-tetramethyl-6,9,tetramethyl-6,9,10-trioxa-tricyclo-[3.3.1.1{3,7}]decyl)-hexan ausgewählt.

Zur Herstellung der Diphosphin-Liganden der Formel I kann man z. B. eine Verbindung mit 2 primären Phosphingruppen mit einem 1,3-Diketon, z. B. 2,4-Pentandion oder substituierten 2,4-Pentandionen, wie Perfluor-2,4-pentandion oder 1,1,1,5,5,5-Hexafluoro-2,4-pentandion, unter Säurekatalyse umsetzen. Die Verbindungen der Formel I werden im Allgemeinen in hoher Reinheit erhalten und können ohne weitere Aufreinigung unmittelbar verwendet werden. Bezüglich geeigneter Reaktionsbedingungen wird auf J. Am. Chem. Soc. 1961, Vol. 83, 3279-3282 und Chem. Com. 1999 (10, 1901-902) sowie die WO 98/42717 verwiesen.

Im Allgemeinen werden unter Hydroformylierungsbedingungen aus den eingesetzten Katalysatoren oder Katalysatorvorstufen katalytisch aktive Spezies der allgemeinen Formel HₓM_{y}(CO)_{z}L_{q} gebildet, worin M für ein Metall der VIII. Nebengruppe des Periodensystems, L für einen Liganden der allgemeinen Formel I und q, x, y, z für ganze Zahlen in Abhängigkeit von der Wertigkeit und Art des Metalls stehen. Die Komplexe können gewünschtenfalls zusätzlich weitere Liganden aufweisen, die vorzugsweise ausgewählt sind unter Halogeniden, Aminen, Carboxylaten, Acetylacetonat, Aryl- und Alkylsulfonaten, Olefinen, Dienen, Cycloolefinen, Nitrilen, stickstoffhaltigen Heterocyclen, Aromaten und Heteroaromaten, Ethern, PF₃, sowie 1-, 2- und mehrzähnigen Phosphin-, Phospinit-, Phosphonit- und Phosphitliganden, die nicht der Formel I entsprechen.

Bei dem Metall der VIII. Nebengruppe handelt es sich vorzugsweise um Cobalt, Ruthenium, Rhodium, Nickel, Palladium, Platin, Osmium oder Iridium und insbesondere um Cobalt, Ruthenium, Iridium, Rhodium, Nickel, Palladium und Platin. Rhodium ist am meisten bevorzugt. Geeignete Quellen der genannten Metalle sind im Allgemeinen deren Verbindungen, z. B. Salze, oder Komplexe.

Nach einer bevorzugten Ausführungsform werden die Hydroformylierungskatalysatoren in situ in dem für die Hydroformylierungsreaktion eingesetzten Reaktor hergestellt. Gewünschtenfalls können die Ligand-Metall-Komplexe jedoch auch separat hergestellt und nach üblichen Verfahren isoliert werden. Zur in-situ-Herstellung kann man z. B. wenigstens einen Ligand der Formel I, eine Verbindung oder einen Komplex eines Metalls der VIII. Nebengruppe, gegenenenfalls wenigstens einen weiteren Liganden und gegebenenfalls ein Aktivierungsmittel in einem inerten Lösungsmittel mit Kohlenmonoxid und Wasserstoff unter Hydroformylierungsbedingungen umsetzen.

Geeignete Rhodiumverbindungen oder -komplexe sind z. B. Rhodium(II)- und Rhodium(III)-Salze, wie Rhodium(III)-chlorid, Rhodium(III)-nitrat, Rhodium(III)-Sulfat, Kaliumrhodiumsulfat, Rhodium(II)- bzw. Rhodium(III)carboxylate, wie Rhodium(II)- und Rhodium(III)-acetat, Rhodium(III)oxid, Salze der Rhodium(III)-säure, Trisammoniumhexachlorobrodat(III) usw. Weiterhin eignen sich Rhodiumkomplexe, wie Rhodiumbiscarbonylacetylacetonat, Acetylacetonatobisethylenrhodium(I) usw. Vorzugsweise werden Rhodiumbiscarbonylacetylacetonat, Rhodiumacetat oder Rhodiumethylhexanoat eingesetzt.

Ebenfalls geeignet sind Rutheniumsalze oder -Verbindungen, geeignete Rutheniumsalze sind beispielsweise Ruthenium(III)-chlorid, Ruthenium(IV)-, Ruthenium(VI)- oder Ruthenium(VIII)oxid, Alkalisalze der Rutheniumsauerstoffsäuren wie K₂RuO₄ oder KRuO₄ oder Komplexverbindungen, wie RuHCL(CO)(PPh₃)₃. Auch können die Metallcarbonyle des Rutheniums, wie Trisrutheniumdodecacarbonyl oder Hexarutheniumoctadecacarbonyl oder Mischformen, in denen CO teilweise durch Triorganophosphine ersetzt sind, wie Ru(CO)₃(PPh₃)₂ verwendet werden.

Geeignete Cobaltverbindungen sind beispielsweise Cobalt(II)-chlorid, Cobalt(II)-sulfat, Cobalt(II)-carbonat, Cobalt(II)-nitrat, deren Amin- oder Hydratkomplexe, Cobaltcarboxylate, wie Cobaltformiat, Cobaltacetat, Cobaltethylhexanoat, Cobaltnaphtanoat, sowie der Cobaltcaprolactamatkomplex. Auch hier können die Carbonylkomplexe des Cobalts wie Dicobaltoctacarbonyl, Tetracobaltdodecacarbonyl und Hexacobalthexadecacarbonyl eingesetzt werden.

Die genannten und weitere geeignete Verbindungen des Cobalts, Rhodiums, Rutheniums und Iridiums sind im Prinzip bekannt und in der Literatur hinreichend beschrieben oder sie können vom Fachmann analog zu den bereits bekannten Verbindungen hergestellt werden. Geeignete Aktivierungsmittel sind z. B. Brönstedsäuren, Lewissäuren, wie z. B. BF₃, AlCl₃, ZnCl₂ und Lewisbasen.

Als Substrat für das hier beschriebene Verfahren kommen prinzipiell alle Verbindungen in Betracht, welche eine oder mehrere Vinyl- oder Vinylidengruppen enthalten. Dazu zählen z. B. C₃-C₂₀-α-Alkene, insbesondere lineare C₃-C₂₀-α-Alkene, wie Propen, 1-Buten, 1-Penten, 1-Hexen, 1-Hepten, 1-Octen, 1-Nonen, 1-Decen, 1-Undecen, 1-Dodecen usw. Ein weiteres bevorzugtes Einsatzmaterial ist Isobuten.

Bevorzugte Einsatzmaterialien sind weiterhin ω-Nitrilo-C₂-C₂₀-alkene, wie Acrylonitril und 4-Pentennitril; und ω-Alkyloxycarbonyl-C₂-C₂₀-alkene, wie Acrylsäurealkylester und 4-Pentensäurealkylester. Geeignet sind weiterhin Vinylaromaten, wie Styrol oder Vinylpyridin.

Die Hydroformylierungsreaktion kann kontinuierlich, semikontinuierlich oder diskontinuierlich erfolgen. Geeignete Reaktoren sind dem Fachmann bekannt und werden z. B. in Ullmann's Enzyklopädie der technischen Chemie, Band 1, 3. Auflage, 1951, S. 743 ff. beschrieben.

Kohlenmonoxid und Wasserstoff werden üblicherweise in Form eines Gemisches, dem sogenannten Synthesegas, eingesetzt. Das molare Verhältnis von Kohlenmonoxid und Wasserstoff beträgt in der Regel 5:95 bis 70:30, bevorzugt 40:60 bis 60:40. Insbesondere wird ein molares Verhältnis von Kohlenmonoxid und Wasserstoff im Bereich von 1:1 eingesetzt.

Die Temperatur bei der Hydroformylierungsreaktion liegt im Allgemeinen in einem Bereich von etwa 80 bis 180°C, vorzugsweise 100 bis 160°C. Die Reaktion wird in der Regel bei dem Partialdruck des Reaktionsgases bei der gewählten Reaktionstemperatur durchgeführt. Im Allgemeinen liegt der Druck in einem Bereich von 5 bis 200 bar, insbesondere 5 bis 30 bar. Die optimale Temperatur und der optimale Druck sind von der eingesetzten ungesättigten Verbindung abhängig.

Die katalytisch aktiven Ligand-Metall-Komplexe lassen sich nach üblichen, dem Fachmann bekannten Verfahren vom Austrag der Hydroformylierungsreaktion abtrennen und können im Allgemeinen, gegebenenfalls nach Aufarbeitung, erneut für die Hydroformylierung eingesetzt werden.

Bei der Hydroformylierung können Lösungsmittel mitverwendet werden, wie die hochsiedenden Folgereaktionsprodukte der Aldehyde, die bei der Hydroformylierung entstehen. Ebenfalls geeignete Lösungsmittel sind aromatische Kohlenwasserstoffe, wie Toluol und Xylol, aliphatische Kohlenwasserstoffe, Ether, wie 2,5,8-Trioxanonan, Diethylether und Anisol, Sulfone, wie Sulfolan, oder Ester, wie 3-Hydroxy-2,2,4-trimethylpentyl-1-isobutyrat (Texanol).

Durch das erfindungsgemäße Verfahren werden im Allgemeinen n-Selektivitäten von mehr als 80%, insbesondere von mehr als 90% erreicht. Die Erfindung wird durch die folgenden nicht einschränkenden Beispiele näher veranschaulicht:

### Beispiele

Die Synthese der Liganden erfolgte gemäß der Beschreibung der WO 98/42717. Die Abkürzung "acac" steht für Acetylacetonat; L:M für das molare Verhältnis von Ligand zu Metall. Die in den Beispielen erhaltenen Reaktionsgemische wurden mittels Gaschromatographie (GIC) analysiert.

### Vergleichsbeispiel 1

### Hydroformylierung von 1-Octen mit 1,3-P,P'-Di(2-phospha-1,3,5,7-tetramethyl-6,9,10-trioxa-tricyclo[3.3.1.1{3,7}]decyl-propan

0,9 mg Rh(CO)₂acac und 1,65 mg 1,3-P,P'-Di(2-phospha-1,3,5,7-tetramethyl-6,9,10-trioxa-tricyclo[3.3.1.1{3,7}]decyl-propan (60 ppm Rh, L:M 1:1) wurden separat in insgesamt 3 g Toluol gelöst, vermischt und bei 100°C mit 10 bar Synthesegas (CO:H₂ = 1:1) begast. Nach 30 min. wurde entspannt, dann wurden 3 g 1-Octen zugegeben und 4 h bei 100°C und 10 bar hydroformyliert. Der Umsatz betrug 94%, die Aldehydselektivität 11%, die Selektivität zu internen Olefinen 89%. Das molare Verhältnis von n-Nonanal zu Isononanal betrug 45:55.

### Vergleichsbeispiel 2

### Hydroformylierung von 1-Octen mit 1,3-P,P'-Di(2-phospha-1,3,5,7-tetramethyl-6,9,10-trioxa-tricyclo[3.3.1.1{3,7}]decyl-propan

0,9 mg Rh(CO)₂acac und 2,47 mg 1,3-P,P'-Di(2-phospha-1,3,5,7-tetramethyl-6,9,10-trioxa-tricyclo[3.3.1.1{3,7}]decyl-propan (60 ppm Rh, L:M = 1,5:1) wurden separat in insgesamt 3 g Toluol gelöst, vermischt und bei 100°C mit 10 bar Synthesegas (CO:H₂ = 1:1) begast. Nach 30 min. wurde entspannt, dann wurden 3 g 1-Octen zugegeben und 4 h bei 100°C und 10 bar hydroformyliert. Der Umsatz betrug 83%, die Aldehydselektivität 12%, die Selektivität zu internen Olefinen 88%. Das molare Verhältnis von n-Nonanal zu Isononanal betrug 43:57.

### Vergleichsbeispiel 3

### Hydroformylierung von 1-Octen mit 1,3-P,P'-Di(2-phospha-1,3,5,7-tetramethyl-6,9,10-trioxa-tricyclo[3.3.1.1{3,7}]decyl-propan

0,9 mg Rh(CO)₂acac und 3,3 mg 1,3-P,P'-Di(2-phospha-1,3,5,7-tetramethyl-6,9,10-trioxa-tricyclo[3.3.1.1{3,7}]decyl-propan (60 ppm Rh, L:M = 2:1) wurden separat in insgesamt 3 g Toluol gelöst, vermischt und bei 100°C mit 10 bar Synthesegas (CO:H₂ = 1:1) begast. Nach 30 min. wurde entspannt, dann wurden 3 g 1-Octen zugegeben und 4 h bei 100°C und 10 bar hydroformyliert. Der Umsatz betrug 83%, die Aldehydselektivität 10%, die Selektivität zu internen Olefinen 90%. Das molare Verhältnis von n-Nonanal zu Isononanal betrug 44:56.

### Vergleichsbeispiel 4

### Hydroformylierung von 1-Octen mit 1,3-P,P'-Di(2-phospha-1,3,5,7-tetramethyl-6,9,10-trioxa-tricyclo[3.3.1.1{3,7}]decyl-propan

0,9 mg Rh(CO)₂acac und 4,9 mg 1,3-P,P'-Di(2-phospha-1,3,5,7-tetramethyl-6,9,10-trioxa-tricyclo[3.3.1.1{3,7}]decyl-propan (60 ppm Rh, L:M = 3:1) wurden separat in insgesamt 3 g Toluol gelöst, vermischt und bei 100°C mit 10 bar Synthesegas (CO:H₂ = 1:1) begast. Nach 30 min. wurde entspannt, dann wurden 3 g 1-Octen zugegeben und 4 h bei 100°C und 10 bar hydroformyliert. Der Umsatz betrug 85%, die Aldehydselektivität 27%, die Selektivität zu internen Olefinen 73%. Das molare Verhältnis von n-Nonanal zu Isononanal betrug 72:28.

### Vergleichsbeispiel 5

### Hydroformylierung von 1-Octen mit 1,3-P,P'-Di(2-phospha-1,3,5,7-tetramethyl-6,9,10-trioxa-tricyclo[3.3.1.1{3,7}]decyl-propan

0,9 mg Rh(CO)₂acac und 6,6 mg 1,3-P,P'-Di(2-phospha-1,3,5,7-tetramethyl-6,9,10-trioxa-tricyclo[3.3.1.1{3,7}]decyl-propan (60 ppm Rh, L:M = 4:1) wurden separat in insgesamt 3 g Toluol gelöst, vermischt und bei 100°C mit 10 bar Synthesegas (CO:H₂ = 1:1) begast. Nach 30 min. wurde entspannt, dann wurden 3 g 1-Octen zugegeben und 4 h bei 100°C und 10 bar hydroformyliert. Der Umsatz betrug 85%, die Aldehydselektivität 25%, die Selektivität zu internen Olefinen 75%. Das molare Verhältnis von n-Nonanal zu Isononanal betrug 75:25.

### Beispiel 6

### Hydroformylierung von 1-Octen mit 1,3-P,P'-Di(2-phospha-1,3,5,7-tetramethyl-6,9,10-trioxa-tricyclo[3.3.1.1{3,7}]decyl-propan

0,9 mg Rh(CO)₂acac und 8,2 mg 1,3-P,P'-Di(2-phospha-1,3,5,7-tetramethyl-6,9,10-trioxa-tricyclo[3.3.1.1{3,71}]decyl-propan (60 ppm Rh, L:M = 5:1) wurden separat in insgesamt 3 g Toluol gelöst, vermischt und bei 100°C mit 10 bar Synthesegas (CO:H₂ = 1:1) begast. nach 30 min. wurde entspannt, dann wurden 3 g 1-Octen zugegeben und 4 h bei 100°C und 10 bar hydroformyliert. Der Umsatz betrug 100%, die Aldehydselektivität 69%, die Selektivität zu internen Olefinen 31%. Das molare Verhältnis von n-Nonanal zu Isononanal betrug 97:3.

### Beispiel 7

### Hydroformylierung von 1-Octen mit 1,3-P,P'-Di(2-phospha-1,3,5,7-tetramethyl-6,9,10-trioxa-tricyclo[3.3.1.1{3,7}]decyl-propan

0,9 mg Rh(CO)₂acac und 16,4 mg 1,3-P,P'-Di(2-phospha-1,3,5,7-tetramethyl-6,9,10-trioxa-tricyclo[3.3.1.1{3,7}]decyl-propan (60 ppm Rh, L:M = 10:1) wurden separat in insgesamt 3 g Toluol gelöst, vermischt und bei 100°C mit 10 bar Synthesegas (CO:H₂ = 1:1) begast. Nach 30 min. wurde entspannt, dann wurden 3 g 1-Octen zugegeben und 4 h bei 100°C bei 10 bar Synthesegas (CO:H₂ = 1:1) begast. Nach 30 min. wurde entspannt, dann wurden 3 g 1-Octen zugegeben und 4 h bei 100°C und 10 bar hydroformyliert. Der Umsatz betrug 80%, die Aldehydselektivität 95%. Das molare Verhältnis von n-Nonanal zu Isononanal betrug 97:3.

Die Beispiele zeigen, dass bei einem molaren Verhältnis von Ligand zu Metall von wenigstens 5 der Anteil des n-Aldehyds an den erhaltenen Aldehyden signifikant höher ist als bei geringeren Ligand-Metall-Verhältnissen.

## Patentansprüche

1. Verwendung eines zweizähnigen Diphosphin-Liganden der allgemeinen Formel I 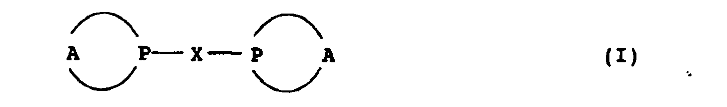 worin
A zusammen mit dem Phosphoratom, an das es gebunden ist, jeweils einen 2-Phospha-tricyclo[3.3.1.1{3,7}]decylrest bildet, in dem gegebenenfalls ein oder mehrere nichtbenachbarte Kohlenstoffatome durch Sauerstoffatome ersetzt sind und der gegebenenfalls substituiert ist, und
X für eine verbrückende Kette mit 1 bis 10 Atomen steht,
und einer Quelle eines Metalls der VIII. Nebengruppe in einem molaren Verhältnis von wenigstens 5 zur selektiven Herstellung von n-Aldehyden in einem Verfahren, bei dem man wenigstens eine Verbindung mit einer Vinyl- oder Vinylidengruppe in Gegenwart der Quelle eines Metalls der VIII. Nebengruppe und des zweizähnigen Diphosphin-Liganden mit Kohlenmonoxid und Wasserstoff umsetzt.

2. Verwendung nach Anspruch 1, wobei X für eine C₁-C₁₀-Alkylenbrücke steht, die eine, zwei, drei oder vier Doppelbindungen aufweisen kann und/oder durch ein, zwei oder drei nichtbenachbarte Heteroatome unterbrochen sein kann und/oder ein-, zwei- oder dreifach mit einem gesättigten oder ungesättigten 3- bis 7-gliedrigen Carbocyclus oder Heterocyclus anelliert sein kann.

3. Verwendung nach Anspruch 1 oder 2, wobei das molare Verhältnis von Disphosphin-Ligand zu Metall wenigstens 8 beträgt.

4. Verwendung nach Anspruch 2 oder 3, wobei A zusammen mit dem Phosphoratom, an das es gebunden ist, für eine Gruppe der allgemeinen Formel II 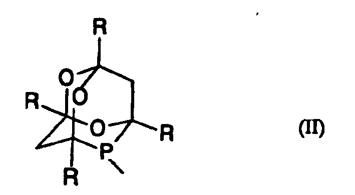 steht, worin die Reste
R unabhängig voneinander für Alkyl, Cycloalkyl, Halogenalkyl, Aryl oder Aralkyl stehen.

5. Verwendung nach Anspruch 4, wobei der Ligand unter 1,2-P,P'-Di(2-phospha-1,3,5,7-tetramethyl-6,9,10-trioxa-tricyclo[3.3.1.1{3,7}]decyl)-ethan, 1,3-P,P'-Di(2-phospha-1,3,5,7-tetramethyl-6,9,10-trioxa-tricyclo[3.3.1.1{3,7}]decyl)-propan und 1,6-P,P'-Di(2-phospha-1,3,5,7-tetramethyl-6,9,10-trioxa-tricyclo[3.3.1.1{3,7}]decyl)-hexan ausgewählt ist.

6. Verwendung nach einem der Ansprüche 1 bis 4, wobei x für eine Kette von drei Atomen steht.

7. Verwendung nach einem der vorhergehenden Ansprüche, wobei die Verbindung mit einer Vinyl- oder Vinylidengruppe unter C₃-C₂₀-α-Alkenen, Isobuten, ω-Nitrilo-C₂-C₂₀-alkenen und ω-Alkyloxycarbonyl-C₂-C₂₀-alkenen ausgewählt ist.

8. Verwendung nach einem der vorhergehenden Ansprüche, wobei es sich bei dem Metall der VIII. Nebengruppe um Rhodium handelt.

## Claims

1. The use of a bidentate diphosphine ligand of the general formula I 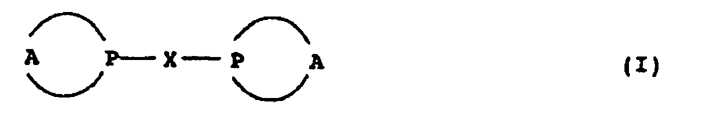 in which
A together with the phosphorus atom to which it is bonded, in each case forms a 2-phosphatricyclo[3.3.1.1{3,7}]decyl radical, in which, if appropriate, one or more non-adjacent carbon atoms have been replaced by oxygen atoms and which is substituted or unsubstituted, and
X is a bridging chain having 1 to 10 carbon atoms,
and of a source of a metal from sub-group VIII in a molar ratio of at least 5 for the selective preparation of n-aldehydes in a process in which at least one compound containing a vinyl or vinylidene group is reacted with carbon monoxide and hydrogen in the presence of the source of a metal from sub-group VIII and in the presence of the bidentate diphosphine ligand.

2. The use according to claim 1, where X is a C₁-C₁₀₋alkylene bridge, which may have one, two, three or four double bonds and/or may be interrupted by one, two or three non-adjacent heteroatoms and/or may be fused to one, two or three saturated or unsaturated 3- to 7-membered carbocyclic or heterocyclic rings.

3. The use according to claim 1 or 2, where the molar ratio between the diphosphine ligand and the metal is at least 8.

4. The use according to claim 2 or 3, where A, together with the phosphorus atom to which it is bonded, is a group of the general formula II 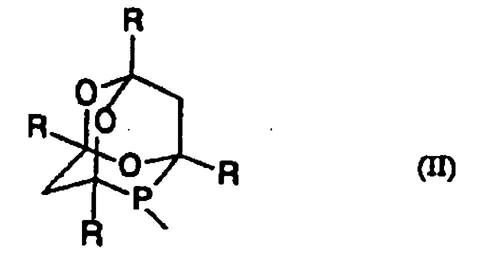 in which the radicals
R independently of one another, are alkyl, cycloalkyl, haloalkyl, aryl or aralkyl.

5. The use according to claim 4, where the ligand is selected from 1,2-P,P'-di(2-phospha-1,3,5,7-tetramethyl-6,9,10-trioxatricyclo[3.3.1.1{3,7}]decyl)ethane, 1,3-P,P'-di(2-phospha-1,3,5,7-tetramethyl-6,9,10-trioxatricyclo[3.3.1.1{3,7}]decyl)propane and 1,6-P,P'-di(2-phospha-1,3,5,7-tetramethyl-6,9,10-trioxatricyclo[3.3.1.1{3,7}]decyl)hexane.

6. The use according to one of claims 1 to 4, where X is a three-atom chain.

7. The use according to one of the preceding claims, where the compound containing a vinyl or vinylidene group is selected from C₃-C₂₀-α-alkenes, isobutene, ω-nitrilo-C₂-C₂₀-alkenes and ω-alkoxycarbonyl-C₂-C₂₀-alkenes.

8. The use according to one of the preceding claims, where the metal from sub-group VIII is rhodium.

## Revendications

1. Utilisation d'un ligand à base de diphosphine bidentate de la formule générale I : 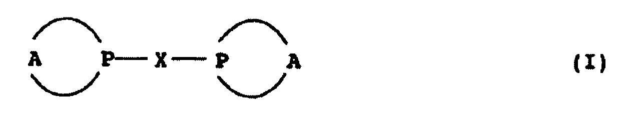 dans laquelle
A forme conjointement avec l'atome de phosphore auquel il est lié chaque fois un radical 2-phospha-tricyclo[3.3.1.1{3,7}]décyle, dans lequel éventuellement un ou plusieurs atomes de carbone non voisins sont remplacés par des atomes d'oxygène et qui est éventuellement substitué, et
X représente une chaîne formant pont comportant 1 à 10 atomes,
et d'une source d'un métal du groupe secondaire VIII dans un rapport molaire d'au moins 5, pour la préparation sélective de n-aldéhydes dans un procédé dans lequel on fait réagir au moins un composé comportant un groupe vinyle ou vinylidène avec du monoxyde de carbone et de l'hydrogène en présence de la source d'un métal du groupe secondaire VIII et du ligand à base de diphosphine bidentate.

2. Utilisation suivant la revendication 1, dans laquelle X représente un pont d'alkylène en C₁-C₁₀ qui peut présenter une, deux, trois ou quatre doubles liaisons et/ou peut être interrompu par un, deux ou trois hétéroatomes non voisins et/ou peut être condensé à une, deux ou trois reprises avec un hétérocycle ou carbocycle à 3 jusqu'à 7 termes, saturé ou insaturé.

3. Utilisation suivant la revendication 1 ou 2, dans laquelle le rapport molaire entre le ligand à base de diphosphine et le métal est d'au moins 8.

4. Utilisation suivant la revendication 2 ou 3, dans laquelle A représente, conjointement avec l'atome de phosphore auquel il est lié, un groupe de la formule générale II : 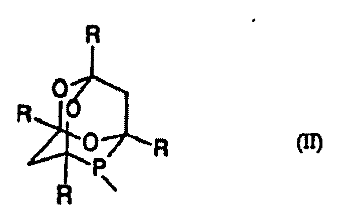 dans laquelle les radicaux
R représentent indépendamment l'un de l'autre un groupe alkyle, cycloalkyle, halogénoalkyle, aryle ou aralkyle.

5. Utilisation suivant la revendication 4, dans laquelle le ligand est choisi parmi du 1,2-P,P'-di(2-phospha-1,3,5,7-tétraméthyl-6,9,10-trioxatricyclo[3.3.1.1{3,7}]décyl)-éthane, du 1,3-P,P'-di(2-phospha-1,3,5,7-tétraméthyl-6,9,10-trioxatricyclo[3.3.1.1{3,7}]décyl)-propane et 1,6-P,P'-di(2-phospha-1,3,5,7-tétraméthyl-6,9,10-trioxatricyclo[3.3.1.1{3,7}]décyl)-hexane.

6. Utilisation suivant l'une des revendications 1 à 4, dans laquelle X représente une chaîne de trois atomes.

7. Utilisation suivant l'une des revendications précédentes, dans laquelle le composé comportant un groupe vinyle ou vinylidène est choisi parmi des α-alcènes en C₃-C₂₀, de l' isobutène, des ω-nitrilo-C₂-C₂₀-alcènes et des ω-alkyloxycarbonyl-C₂-C₂₀-alcènes.

8. Utilisation suivant l'une des revendications précédentes, dans laquelle, pour ce qui concerne le métal du groupe secondaire VIII, il s'agit de rhodium.
